# EUROPEAN PATENT SPECIFICATION

(11) **EP 0 453 914 B1**
(45) Date of publication and mention of the grant of the patent: **28.12.1994**
(21) Application number: 91105919.4
(22) Date of filing: 13.04.1991
(51) Int. Cl.: C07C 263/20

(54) **Melt crystallization process for preparing high trans, trans-isomer containing 4,4'-diisocyanato dicyclohexylmethane**
Kristallisationsverfahren aus der Schmelze zur Herstellung von 4,4'-Diisocyanatodicyclohexylmethan mit einem hohen Anteil des trans,trans-Isomeren
Procédé de cristallisation à l'état fondu pour la préparation de 4,4'-diisocyanato dicyclohexylméthane à haute teneur en trans,trans isomère

(30) Priority: 24.04.1990 US 513698; 13.02.1991 US 654862
(43) Date of publication of application: 30.10.1991
(73) Proprietor: MILES INC., Pittsburgh, PA 15219-2502 (US)
(72) Inventor: Seneker, Stephen D., Paden City, WV 26159 (US); Dunlap, Kenneth L., New Martinsville, WV 26155 (US); Lowery, Michael K., Pittsburgh, PA 15243 (US); Potter, Terry A., New Martinsville, WV 26155 (US)
(74) Representative: Weber, Gerhard

(56) References cited:
- DE-A- 1 568 898
- US-A- 4 983 763

## Description

4,4'-diisocyanato dicyclohexylmethane ("PICM") is a cycloaliphatic diisocyanate of low volatility. PICM and other aliphatic isocyanates are useful in the preparation of non-discoloring polyurethanes. In general, such isocyanates are reacted with glycols and/or polyols and chain extenders and/or cross linkers. Such isocyanates are particularly useful in the preparation of polyurethane coatings and elastomers.

PICM and the diamine precursor, 4,4'-diamino-dicyclohexylmethane ("PACM"), exist in three stereoisomeric forms (i.e., trans,trans; cis,trans; and cis,cis) as described, for example, in U.S. Patents 2,606,925, and 3,789,032, Canadian Patents 961,049 and 971,184, and British Patent 1,220,715. Commercial grades of PACM normally contain all three isomers.

The most direct method of producing PICM is to first hydrogenate diamino diphenylmethane to form a mixture of the stereoisomers of PACM, and to then phosgenate the mixture. When the synthesis of PICM is conducted using readily available mixtures of stereoisomers of PACM (such as the equilibrium mixture described in U.S. 3,155,724), the PICM obtained is a slurry at normal operating temperatures, having a melting point of about 58°C, which corresponds to a trans,trans-isomer content of about 54%. Various PICM mixtures are known in the art which have trans,trans-isomer contents of from about 18 to about 55% by weight. In addition, the art has recognized an advantage in utilizing high trans,trans-isomer PICM in producing elastomers (see, U.S. Patent 3,789,032).

In order to prepare PICM of relatively high trans,trans-isomer content, the art has generally used a PACM having a relatively high trans,trans-isomer content in the phosgenation reaction. Various methods are known for treating PACM to obtain the requisite high trans,trans-isomer content. Crystallization techniques have been described in the art. See, e.g., U.S. Patents 2,494,563, 3,153,088, 3,384,661 and 3,393,236. The crystallization of PACM suffers from various disadvantages. PACM readily forms a precipitant when exposed to carbon dioxide, causing problems in filtering and contamination of the crystals (see, U.S. Patent 2,494,563, column 3, lines 26-29, and column 4, lines 72-75). In addition, PACM is generally difficult to crystallize since it will easily form a supercooled liquid. The prior art has overcome this problem by adding seed crystals (U.S. Patent 2,494,563), by lowering the viscosity by using an inert solvent (U.S. Patents 2,494,563, 3,153,088, 3,393,236 and 3,384,661), or by forming an adduct of PACM that crystallizes better, such as the hydrate (U.S. Patent 3,153,088) or the alcoholate (U.S. Patent 3,384,661). Such an adduct must be treated to remove water or alcohol before phosgenating to PICM.

It is known to separate the trans,trans-isomer from an industrial mixtures of PICM having a trans,trans content of from 18 to 24% by weight by fractional crystallization followed by washing with cold hexane and then vacuum distillation. The mixture was cooled to 10°C until crystallization took place and the solid fraction (the trans,trans-isomer) was removed by filtration in a nitrogen atmosphere. The residue was washed with cold hexane and stored under a nitrogen atmosphere. The filtrate was recooled and any new solids removed. See, Byrne et al, "A Study of Aliphatic Polyurethane Elastomers Prepared From Diisocyanate Isomer Mixtures," Rubber Chemistry and Technology, Vol. 58, 1985, pages 985-996, and Wong et al, "Structure-Property Relationships of Transparent Aliphatic Polyurethane Elastomers From the Geometric Isomers of Methylene bis(4-Cyclohexyl Isocyanate)," Advances in Urethane Science and Technology, Vol. 9, 1984, pages 77-101. The major disadvantage of this method is that solvent is required. In addition, the yield of the trans,trans-isomer is poor. Finally, cooling below room temperature is expensive on an industrial scale.

### Description of the Invention

The present invention is directed to a relatively simple process for preparing a PICM containing a relatively high amount of the trans,trans-isomer, which process overcomes the problems associated with the prior art. More particularly, the present invention is directed to a process for the preparation of a 4,4'-diisocyanato dicyclohexylmethane containing at least 90% by weight, and preferably at least 94% by weight, of the trans,trans-isomer comprising:
(a) melting a mixture of 4,4'-diisocyanato dicyclohexylmethane isomers containing at least 30% by weight, and preferably from 45 to 55% by weight, of the trans,trans-isomer,
(b) cooling the melted mixture to a temperature of from 0 to 25°C to form:
   (1) a liquid phase which contains from 12 to 25% by weight of the trans,trans-isomer, and
   (2) a solid phase which contains from 70 to 85% by weight of the trans,trans-isomer,
(c) removing said liquid phase, which is a commercially viable product itself,
(d) heating said solid phase to a temperature of from 60 to the melting point of the pure trans,trans-isomer, which is about 83°C (preferably over a period of from 3 to 5 hours and while maintaining that temperature for a period of from 1 to 2 hours), while removing that portion of said solid phase which has melted, with the melted material containing from 35 to 65% by weight, and preferably from 45 to 55% by weight, of the trans,trans-isomer, and
(e) recovering that portion of said solid phase which has not melted by heating the said remaining portion to a temperature of at least 90°C with the resultant product containing at least 90% by weight, and preferably at least 94% by weight, of the trans,trans-isomer.

In one particularly preferred embodiment, the melted material of step (d) is recycled to step (a).

The presently preferred process comprises:
(a) melting a mixture of 4,4'-diisocyanato dicyclohexylmethane isomers containing at least 30% by weight, and preferably from 45 to 55% by weight, of the trans,trans-isomer,
(b) cooling the melted mixture to a temperature of from 40 to 45°C for a time sufficient to allow crystallization to occur (generally for two to eight hours) and then subsequently cooling to a temperature of from 0 to 25°C to form:
   (1) a liquid phase which contains from 12 to 25% by weight of the trans,trans-isomer, and
   (2) a solid phase which contains from 70 to 85% by weight of the trans,trans-isomer,
(c) removing said liquid phase, which is a commercially viable product,
(d1) heating said solid phase to a temperature of from 65 to 70°C (and, preferably, maintaining that temperature for a period of from 60 to 120 minutes),
(d2) removing that portion of said solid phase which has melted, and
(e) recovering that portion of said solid phase which has not melted during step (d1) by heating the said remaining portion to a temperature of about 100°C with the resultant product containing at least 90% by weight, and preferably at least 94% by weight, of the trans,trans-isomer.

In a particularly preferred embodiment, the liquid phase of step (c) and the melted product removed during step (d2) are combined to make a liquid product.

Substantially any mixture of PICM containing at least 30% by weight, and preferably containing from 45 to 55% by weight, of the trans,trans-isomer can be used in step (a) of the process of the invention. It is generally preferred to utilize commercially available mixtures which typically contain about 50% by weight of the trans,trans-isomer. The particular mixture selected is melted, typically by heating to a temperature of from 60 to 83°C, preferably for a period of from 120 to 180 minutes. The melted mixture is then cooled to a temperature of from 0 to 25°C to form liquid and solid phases having the trans,trans-isomer contents noted above. Preferably, the melt is cooled to the requisite temperature in from 2 to 8 hours, and is held at that temperature for from 60 to 120 minutes.

The liquid and solid phases formed by cooling the melt can be separated by substantially any technique known in the art, such as, for example, filtration, decanting, centrifugation, or merely draining the liquid. The solid phase is then heated to a temperature equal to or less than the melting point of the pure product. This process is known to those familiar with the art as "sweating". The solid phase which has not melted is then recovered by heating to about 100°C.

The invention is not limited to a batch process and can also be performed in a continuous fashion through a series of heating and cooling cycles, e.g., as in a continuous crystallizer.

The invention is further illustrated but is not intended to be limited by the following examples in which all parts and percentages are by weight unless otherwise specified.

### EXAMPLES

### EXAMPLE 1

3300 parts of a 4,4'-diisocyanato dicyclohexylmethane containing 50% by weight of the trans,trans-isomer were charged to a one gallon glass jar and completely melted at 80°C. The melt was then allowed to cool to room temperature (22°C) and kept at that temperature for 16 hours. The liquid and solid phases were separated at room temperature by draining the liquid through a valve at the bottom of the vessel. The liquid phase contained 22.3% by weight of the trans,trans-isomer, while the solid phase contained 80.9% by weight of the trans,trans-isomer. The weight percent yields of liquid and solid phases were 53 and 47% respectively. The solid phase consisted of a three-dimensional matrix of crystals in the vessel.

The temperature of the vessel which contained the 1551 parts of the solid phase (i.e., 47% of 3300) was increased to 40°C and held at that temperature for 2 hours. The fraction of the product that melted was drained. Then, the temperature was increased in 5°C increments every two hours until a temperature of 80°C was reached. The liquid generated was continuously drained during the heating step. When 80°C was reached, the composition of the solid contained 93.8% by weight of the trans,trans-isomer. The weight percent yield with respect to the 1551 parts of the solid phase was 60%. The solid phase was removed from the glass jar by melting at a temperature of about 90°C.

### EXAMPLE 2

869 parts of a 4,4-diisocyanato dicyclohexylmethane containing 50 % by weight of the trans,trans-isomer were charged to a jacketed column equipped with heating and cooling means. The material was completely melted at 80°C and the melt was then allowed to cool to 45°C and kept at that temperature for 8 hours. The solid and liquid phases were then cooled to 25°C and held at that temperature for 4 hours. The liquid and solid phases were separated at this temperature by draining the liquid through a valve at the bottom of the column thereby obtaining 458 parts of liquid product. The solid phase was then heated to 45-50°C with 68.5 parts of melted product being removed. After two hours, the column was heated to 65°C while removing an additional 25.5 parts of melted product. The liquid fractions were combined to form a liquid product that contained 24.2% by weight of the trans,trans-isomer. The remaining solid product (316 parts), which contained more than 90% by weight of the trans,trans-isomer was removed by heating the column to 100°C.

## Claims

1. A process for the preparation of a 4,4'-diisocyanato dicyclohexylmethane containing at least 90% by weight of the trans,trans-isomer comprising:
(a) melting a mixture of 4,4'-diisocyanato dicyclohexylmethane isomers containing at least 30% by weight of the trans,trans-isomer,
(b) cooling the melted mixture to a temperature of from 0 to 25°C to form:
(1) a liquid phase which contains from 12 to 25% by weight of the trans,trans-isomer, and
(2) a solid phase which contains from 70 to 85% by weight of the trans,trans-isomer,
(c) removing said liquid phase,
(d) heating said solid phase to a temperature of from 60 to 83°C and removing that portion of said solid phase which has melted, with the melted material containing from 35 to 65% by weight, of the trans,trans-isomer, and
(e) recovering that portion of said solid phase which has not melted by heating the said remaining portion to a temperature of at least 90°C with the resultant product containing at least 90% by weight of the trans,trans-isomer.

2. The process of Claim 1, wherein the mixture of step (a) contains from 45 to 55% by weight of the trans,trans-isomer.

3. The process of Claim 1, wherein the melted mixture removed in step (d) contains from 45 to 55% by weight of the trans,trans-isomer.

4. The process of Claim 1, wherein the product recovered in step (e) contains at least 94% by weight of the trans,trans-isomer.

5. The process of Claim 1, wherein the melted material of step (d) is returned to step (a).

6. A process for the preparation of a 4,4'-diisocyanato dicyclohexylmethane containing at least 90% by weight of the trans,trans-isomer comprising:
(a) melting a mixture of 4,4'-diisocyanato dicyclohexylmethane isomers containing at least 30% by weight of the trans,trans-isomer,
(b) cooling the melted mixture to a temperature of from 40 to 45°C for a period of time sufficient to allow crystallization to occur, and then subsequently cooling to a temperature of from 0 to 25°C to form:
(1) a liquid phase which contains from 12 to 25% by weight of the trans,trans-isomer, and
(2) a solid phase which contains from 70 to 85% by weight of the trans,trans-isomer,
(c) removing said liquid phase,
(d1) heating said solid phase to a temperature of from 65 to 70°C,
(d2) removing that portion of said solid phase which has melted, and
(e) recovering that portion of said solid phase which has not melted during step (d1) by heating the said remaining portion to a temperature of about 100°C with the resultant product containing at least 90% by weight of the trans,trans-isomer.

7. The process of Claim 6, wherein the mixture of step (a) contains from 45 to 55% by weight of the trans,trans-isomer.

8. The process of Claim 6, wherein the product recovered in step (e) contains at least 94% by weight of the trans,trans-isomer.

9. The process of Claim 6, wherein the liquid phase of step (c) and the melted product removed during step (d2) are combined to make a liquid product.

## Patentansprüche

1. Verfahren zur Herstellung von 4,4'-Diisocyanatodicyclohexylmethan, das wenigstens 90 Gew.-% des trans,trans-Isomers enthält, umfassend
(a) das Schmelzen eines Gemisch der 4,4'-Diisocyanatodicyclohexylmethan-Isomeren, das wenigstens 30 Gew.-% des trans,trans-Isomers enthält,
(b) das Kühlen des geschmolzenen Gemischs auf eine Temperatur von 0 °C bis 25 °C zur Bildung
(1) einer flüssigen Phase, die 12 bis 25 Gew.-% des trans,trans-Isomers enthält, und
(2) einer festen Phase, die 70 bis 85 Gew.-% des trans,trans-Isomers enthält,
(c) das Entfernen der flüssigen Phase,
(d) das Erhitzen der festen Phase auf eine Temperatur von 60 °C bis 83 °C und das Entfernen des Teils jener festen Phase, der geschmolzen ist, wobei das geschmolzene Material 35 bis 65 Gew.-% des trans,trans-Isomers enthält, und
(e) das Isolieren des Teils jener festen Phase, der nicht geschmolzen ist, durch Erhitzen des verbleibenden Teils auf eine Temperatur von wenigstens 90 °C, wobei das resultierende Produkt wenigstens 90 Gew.-% des trans,trans-Isomers enthält.

2. Verfahren nach Anspruch 1, worin das Gemisch aus Schritt (a) 45 bis 55 Gew.-% des trans,trans-Isomers enthält.

3. Verfahren nach Anspruch 1, worin das in Schritt (d) entfernte geschmolzene Gemisch 45 bis 55 Gew.-% des trans,trans-Isomers enthält.

4. Verfahren nach Anspruch 1, worin das in Schritt (e) isolierte Produkt wenigstens 94 Gew.-% des trans,trans-Isomers enthält.

5. Verfahren nach Anspruch 1, worin das geschmolzene Material des Schrittes (d) zu dem Schritt (a) zurückgeführt wird.

6. Verfahren zur Herstellung von 4,4'-Diisocyanatodicyclohexylmethan, das wenigstens 90 Gew.-% des trans,trans-Isomers enthält, umfassend
(a) das Schmelzen eines Gemischs der 4,4'-Diisocyanatodicyclohexylmethan-Isomeren, das wenigstens 30 Gew.-% des trans,trans-Isomers enthält,
(b) das Kühlen des geschmolzenen Gemischs auf eine Temperatur von 40 °C bis 45 °C während einer Zeitspanne, die ausreicht, um das Stattfinden einer Kristallisation zu erlauben, und anschließend das Abkühlen auf eine Temperatur von 0 °C bis 25 °C zur Bildung
(1) einer flüssigen Phase, die 12 bis 25 Gew.-% des trans,trans-Isomers enthält, und
(2) einer festen Phase, die 70 bis 85 Gew.-% des trans,trans-Isomers enthält,
(c) das Entfernen der flüssigen Phase,
(d1) das Erhitzen der festen Phase auf eine Temperatur von 65 °C bis 70 °C,
(d2) das Entfernen des Teils jener festen Phase, der geschmolzen ist, und
(e) das Isolieren des Teils jener festen Phase, der nicht während des Schrittes (d1) geschmolzen ist, durch Erhitzen des verbleibenden Teils auf eine Temperatur von etwa 100 °C, wobei das resultierende Produkt wenigstens 90 Gew.-% des trans,trans-Isomers enthält.

7. Verfahren nach Anspruch 6, worin das Gemisch aus Schritt (a) 45 bis 55 Gew.-% des trans,trans-Isomers enthält.

8. Verfahren nach Anspruch 6, worin das in Schritt (e) isolierte Produkt wenigstens 94 Gew.-% des trans,trans-Isomers enthält.

9. Verfahren nach Anspruch 6, worin die flüssige Phase aus Schritt (c) und das während des Schrittes (d2) entfernte geschmolzene Produkt zu einem flüssigen Produkt kombiniert werden.

## Revendications

1. Procédé pour la préparation d'un 4,4'-diisocyanatodicyclohexylméthane contenant au moins 90 % en poids de l'isomère trans,trans, comprenant les étapes suivantes :
(a) on fait fondre un mélange d'isomères de 4,4'-diisocyanatodicyclohexylméthane contenant au moins 30 % en poids de l'isomère trans,trans,
(b) on refroidit le mélange fondu à une température de 0 à 25°C pour former :
(1) une phase liquide qui contient de 12 à 25 % en poids de l'isomère trans,trans et
(2) une phase solide qui contient de 70 à 85 % en poids de l'isomère trans,trans,
(c) on sépare ladite phase liquide,
(d) on chauffe ladite phase solide à une température de 60 à 83°C et on sépare la portion de ladite phase solide qui a fondu, le produit fondu contenant de 35 à 65 % en poids de l'isomère trans,trans et
(e) on récupère la portion de ladite phase solide qui n'a pas fondu en chauffant ladite portion restante à une température d'au moins 90°C, le produit résultant contenant au moins 90 % en poids de l'isomère trans,trans.

2. Procédé selon la revendication 1, dans lequel le mélange de l'étape (a) contient de 45 à 55 % en poids de l'isomère trans,trans.

3. Procédé selon la revendication 1, dans lequel le mélange fondu séparé dans l'étape (d) contient de 45 à 55 % on poids de l'isomère trans,trans.

4. Procédé selon la revendication 1, dans lequel le produit récupéré dans l'étape (e) contient au moins 94 % en poids de l'isomère trans,trans.

5. Procédé selon la revendication 1, dans lequel le produit fondu de l'étape (d) est renvoyé à l'étape (a).

6. Procédé pour la préparation d'un 4,4'-diisocyanatodicyclohexylméthane contenant au moins 90 % en poids de l'isomère trans,trans comprenant les étapes suivantes :
(a) on fait fondre un mélange d'isomères de 4,4'-diisocyanatodicyelohexylméthane contenant au moins 30 % en poids de l'isomère trans,trans,
(b) on refroidit le mélange fondu à une température de 40 à 45°C pendant une durée suffisante pour que la cristallisation ait lieu et on refroidit ensuite à une température de 0 à 25°C pour former :
(1) une phase liquide qui contient de 12 à 25 % en poids de l'isomère trans,trans et
(2) une phase solide qui contient de 70 à 85 % en poids de l'isomère trans,trans,
(c) on sépare ladite phase liquide,
(d1) on chauffe ladite phase solide à une température de 65 à 70°C,
(d2) on sépare la portion de ladite phase solide qui a fondu, et
(e) on récupère la portion de ladite phase solide qui n'a pas fondu pendant l'étape (d1) en chauffant ladite portion restante à une température d'environ 100°C, le produit résultant contenant au moins 90 % en poids de l'isomère trans,trans.

7. Procédé selon la revendication 6, dans lequel le mélange de l'étape (a) contient de 45 à 55 % en poids de l'isomère trans,trans.

8. Procédé selon la revendication 6, dans lequel le produit récupéré dans l'étape (e) contient au moins 94 % en poids de l'isomère trans,trans.

9. Procédé selon la revendication 6, dans lequel la phase liquide de l'étape (c) et le produit fondu séparé pendant l'étape (d2) sont combinés pour faire un produit liquide.
